(19) Europäisches Patentamt
European Patent Office
Office européen des brevets

(11) Veröffentlichungsnummer: **0 246 194 B1**

# (12) EUROPÄISCHE PATENTSCHRIFT

(45) Veröffentlichungstag der Patentschrift: **30.10.91**

(51) Int. Cl.$^5$: **C07F 15/00**, C07C 29/136, B01J 31/18

(21) Anmeldenummer: **87810294.6**

(22) Anmeldetag: **11.05.87**

## (54) Iridiumkomplexe und deren Verwendung.

(30) Priorität: **16.05.86 CH 1988/86**

(43) Veröffentlichungstag der Anmeldung:
**19.11.87 Patentblatt 87/47**

(45) Bekanntmachung des Hinweises auf die Patenterteilung:
**30.10.91 Patentblatt 91/44**

(84) Benannte Vertragsstaaten:
**BE CH DE FR GB IT LI NL**

(56) Entgegenhaltungen:
**JP-A- 5 920 294**

**INORGANICA CHIMICA ACTA, Band 73, 1983, Lausanne R. USON et al. "Cationic iridium (I) complexes with 1,5-cyclooctadiene and nitrogen ligands" Seiten 275-279**

**JOURNAL OF ORGANOMETALLIC CHEMISTRY; Band 222, 1981, Lausanne G.ZASSINOVICH ET AL: "Enantioselective transfer hydrogenation catalyzed by iridium (I) complexes with nitrogen donor ligands" Seiten 323-329**

(73) Patentinhaber: **CIBA-GEIGY AG**
**Klybeckstrasse 141**
**CH-4002 Basel(CH)**

(72) Erfinder: **Kaschig, Jürgen, Dr.**
**Rötebuckweg 30**
**W-7800 Freiburg(DE)**

EP 0 246 194 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die Erfindung betrifft optisch aktive, kationische Iridium(I)-Komplexe mit asymmetrischen 1,2-Diaminliganden und einem Dienliganden, ein Verfahren zu deren Herstellung und deren Verwendung als enantioselektive Katalysatoren.

G. Zassinovich et al. beschreiben im Journal of Organometallic Chemistry, 222, S. 323-329 (1981) kationische Iridium(I)-Komplexe mit einem 1,5-Cyclooctadienliganden und einem 2-Pyridinaliminliganden, der am Imin-N-Atom durch optisch aktives α-Phenylethyl oder 3-Pinanmethyl substituiert ist. Sie wirken als enantioselektive homogene Katalysatoren bei der Transferhydrierung von prochiralen Ketonen mit Isopropanol. Bei der Reaktion werden zwar hohe Ausbeuten erzielt, aber die optische Ausbeute (Enantiomerenüberschuss) ist relativ gering.

Kationische Iridium(I)-Komplexe mit einem 1,5-Cyclooctodienliganden und einem racemischen 1,2-Diphenylethylendiaminliganden sind aus R. Urson et al, Inorganica Chimica Acta, 73 (1983), S. 275-279 bekannt. Es wird auch erwähnt, dass diese Iridiumkomplexe als Katalysatoren für die Transferhydrierung von Acetophenon und Cyclohexen mit Isopropanol geeignet sind. Die Ausbeuten sind relativ gering und von einer Anreicherung optischer Isomerer wird nichts erwähnt.

Es wurde gefunden, dass man hohe Ausbeuten und eine hohe Anreicherung optischer Isomerer erzielen kann, wenn die Iridiumkomplexe asymetrische diprimäre 1,2-Diaminliganden enthalten.

Ein Gegenstand der Erfindung sind optisch aktive Iridiumkomplexe der Formel I

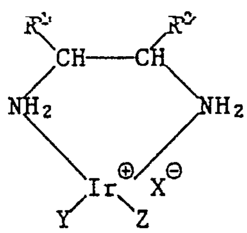

(I),

worin die

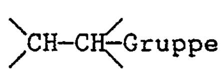

mindestens ein chirales C-Atom enthält, $R^1$ und $R^2$ je Phenyl oder $R^1$ und $R^2$ zusammen unsubstituiertes oder durch $C_1$-$C_4$-Alkyl substituiertes $-(CH_2)4-$ bedeuten, oder $R^1$ für $C_1$-$C_{12}$-Alkyl, unsubstituiertes oder durch $C_1$-$C_4$-Alkyl substituiertes Cycloalkyl mit 5 oder 6 Ring-C-Atomen, $C_6$-$C_{10}$-Aryl oder $C_7$-$C_{12}$-Aralkyl oder $C_8$-$C_{14}$-Alkaralkyl und $R^2$ für H stehen, $X^\ominus$ das Anion einer einbasischen anorganischen oder organischen Säure ist, und Y und Z je Ethylen oder Y und Z zusammen ein offenkettiges oder cyclisches Dien mit 6 bis 10 C-Atomen, dessen Diengruppen über 1 oder 2 C-Atome verbunden sind, bedeuten.

Optisch aktiv bedeutet, dass mindestens ein chirales C-Atom überwiegend in S- oder R-Konfiguration vorliegt.

Bei $X^\ominus$ als Anion einer einbasischen anorganischen oder organischen Säure kann es sich zum Beispiel um $F^\ominus$, $Cl^\ominus$, $Br^\ominus$, $ClO_4^\ominus$, $NO_3^\ominus$, $BrO_3^\ominus$, $HSO_4^\ominus$, $H_2PO_3^\ominus$, $H_2PO_4^\ominus$, $BF_4^\ominus$, $PF_6^\ominus$, $SbF^\ominus$, $AsF_6^\ominus$, $SbCl_6^\ominus$, $SbCl_5F^\ominus$, $HCOO^\ominus$, $CH_3COO^\ominus$, $CCl_3COO^\ominus$, $CF_3COO^\ominus$, $CH_3SO_3^\ominus$, $CCl_3SO_3^\ominus$, $CF_3SO_3^\ominus$, Phenyl-$SO_3^\ominus$ oder p-Toluyl-$SO_3^\ominus$ handeln. In einer bevorzugten Ausführungsform stellt $X^\ominus$ $BF_4^\ominus$, $ClO_4^\ominus$, $CF_3SO_3^\ominus$, $PF_6^\ominus$ dar; besonders bevorzugt ist $X^\ominus$ $BF_4^\ominus$.

Y und Z stehen bevorzugt je für Ethylen oder Y und Z stellen zusammen bevorzugt ein Dien mit 6 bis 8 C-Atomen dar, dessen Diengruppen insbesondere über 2 C-Atome verbunden sind. In einer bevorzugten Ausführungsform stehen Y und Z je für Ethylen oder Y und Z zusammen für 1,5-Cycloactadien, Norbornadien oder 1,5-Hexadien.

Bei dem Substituenten $C_1$-$C_4$-Alkyl kann es sich um Methyl, Ethyl, n-Propyl, i-Propyl und Butyl handeln. Bevorzugt ist Methyl.

In einer bevorzugten Ausführungsform stehen $R^1$ und $R^2$ je für Phenyl oder zusammen für $-(CH_2)_4-$.

$R^1$ kann als Alkyl lineares oder verzweigtes $C_1$-$C_{12}$-Alkyl, bevorzugt $C_1$-$C_6$-Alkyl sein. Beispiele sind Methyl, Ethyl, n-Propyl, i-Propyl, n-, i- und t-Butyl, n-Pentyl, 2-Methylbut-1-yl, Hexyl, Pentyl, Octyl, 2-Ethylhex-1-yl, Nonyl, Decyl, Undecyl und Dodecyl. In einer bevorzugten Ausführungsform stellt $R^1$ als Alkyl Methyl, Ethyl, n-oder i-Propyl oder n-, i- oder t-Butyl dar. $R^1$ als Cycloalkyl ist bevorzugt Cyclopentyl oder Cyclohexyl. $R^1$ als Aryl stellt bevorzugt Phenyl oder Naphthyl dar. $R^1$ ist als Aralkyl kann z.B. Benzyl, α- oder ß-Phenylethyl, 2-Phenylprop-1-yl, 3-Phenylprop-1-yl, 4-Phenyl-but-1-yl oder Phenylhexyl sein. $R^1$ als Alkaralkyl ist bevorzugt $C_8$-$C_{14}$-Alkaralkyl und kann z.B. Methylbenzyl, Ethylbenzyl, Propylbenzyl, Hexylbenzyl oder α- oder ß-(Methylphenyl)ethyl sein.

In einer bevorzugten Ausführungsform ist $R^1$ $C_1$-$C_4$-Alkyl, Cyclopentyl, Cyclohexyl, Phenyl, Benzyl, α-Phenylethyl oder Methylbenzyl. Eine bevorzugte Untergruppe der Iridiumkomplexe der Formel I sind solche, in denen $X^{\ominus}$ $BF_4$ und Y und Z zusammen Cycloactadien bedeuten.

Die Iridiumkomplexe der Formel I können nach an sich bekannten Verfahren [siehe Inorganica Chimica Acta 73 (1983), S. 275-279] erhalten werden, indem man $[(\text{Acetonitril})_2(YZ)]IrX$, worin X, Y und Z die in Anspruch 1 angegebene Bedeutung haben, mit einem optisch aktiven Diamin der Formel II

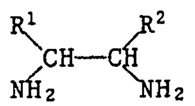

(II),

worin $R^1$ und $R^2$ die zuvor angegebene Bedeutung haben, umsetzt. Die Herstellung des Acetonitrilkomplexes ist dort ebenfalls beschrieben. Die zur Herstellung des Acetonitrilkomplex verwendeten Komplexe $[IrCl(YZ)]_2$ sind z.B. durch die Umsetzung von Dichlorotetrabis(alken)di-ridium(I) (Alken z.B. Cycloocten) mit Ethylen oder einem Dien YZ erhältlich.

Die Umsetzungen werden im allgemeinen bei Temperaturen von -10 bis 30 °c in einem inerten Lösungsmittel und unter Luftausschluss durchgeführt. Geeignete inerte Lösungsmittel sind z.B. Kohlenwasserstoffe wie Benzol, Toluol, Xylol, Petrolether, Hexan, Cyclohexan, Methylcyclohexan; und Ether wie z.B. Diethylether, Dibutylether, Tetrahydrofuran und Dioxan sowie halogenierte Kohlenwasserstoffe, z.B. Chloroform, Methylenchlorid und Chlorbenzol. Zur Herstellung von Salzen der Formel I mit Anionen einbasischer anorganischer oder organischer Säuren können die Salze der Formel I entweder direkt nach der Reaktion oder nach der Isolierung und Reinigung und dem erneuten Lösen in polaren Lösungsmitteln (z.B. Alkoholen, Ethern oder Ketonen, gegebenenfalls unter Zusatz von Wasser) mit einem Alkalisalz $M^{\oplus}X'^{\ominus}$ umgesetzt und danach isoliert werden. $X'^{\ominus}$ bedeutet ein von $X^{\ominus}$ verschiedenes Anion einer einbasischen oder organischen Säure, $M^{\oplus}$ steht bevorzugt für Natrium. Die erfindungsgemässen Iridiumkomplexe sind kristallin und können durch Filtration isoliert und durch Umkristallisation gereinigt werden.

Optisch aktive 1,2-Diamine der Formel II sind bekannt, teilweise käuflich oder sie sind nach bekannten Verfahren und klassischer Racematspaltung herstellbar. (R)-1,2-Diaminopropan und (R,R)-1,2-Diaminocyclohexan sind käuflich. (R,R)-1,2-Diamino-1,2-diphenylethan ist in der Literatur beschrieben. Optisch aktive 1,2-Diamine der Formel II, worin $R^2$ H bedeutet, können nach folgendem Verfahren aus optisch aktiven α-Aminocarbonsäuren hergestellt werden ($Z^1$ ist $-COOCH_2C_6H_5$, * steht für überwiegend S- oder R-Konfiguration):

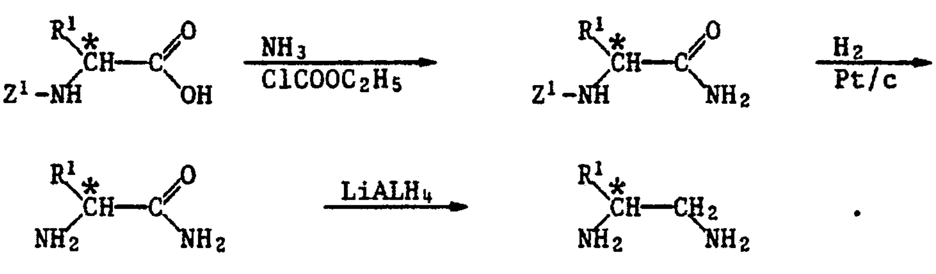


Beispiele für solche Diamine sind (R)- und (S)-1-Methyl-1,2-diaminoethan, (R)- und (S)-1-Ethyl-1,2-diaminoethan, (R)- und (S)-1-(n-Propyl)-1,2-diaminoethan, (R)- und (S)-1-(i-Propyl)-1,2-diaminoethan, (R)- und (S)-1-(n-Butyl)-1,2-diaminoethan, (R)- und (S)-1-(i-Butyl)-1,2-diaminoethan, (R)- und (S)-1-(t-Butyl)-1,2-diaminoethan, (R)- und (S)-1-Phenyl-1,2-diaminoethan, (R)- und (S)-1-Benzyl-1,2-diaminoethan.

Ein weiterer Gegenstand der Erfindung ist die Verwendung der erfindungsgemässen Iridiumkomplexe als enantioselektive homogene Katalystoren für die Transferhydrierung insbesondere prochiraler Ketone mit sekundären Alkoholen. Als sekundärer Alkohol ist besonders Isopropanol geeignet. Die Reaktion wird vorteilhaft unter Sauerstoffausschluss bei erhöhter Temperatur durchgeführt. Zweckmässig wird der verwendete sekundäre Alkohol als Lösungsmittel verwendet. Die Katalysatorkonzentration beträgt bevorzugt $10^{-2}$ bis $10^{-5}$ Mol/l, bezogen auf das Reaktionsvolumen. Die Reaktion wird bevorzugt in Gegenwart einer Base, besonders NaOH, durchgeführt.

Die nachfolgenden Beispiele erläutern die Erfindung näher. Die Bestimmung des Enantiomerenüberschusses (ee) erfolgt nach Mosher [J. Org. Chem. 34, S. 2543 (1969)].

Beispiel 1-6: Unter Argon-Schutzgas werden 0,469 g (1.0 mmol) Bis(acetonitril)(cycloocta-1,5-dien)-iridiumtetrafluorborat in 15 ml Dichlormethan gelöst. Bei Raumtemperatur und unter Rühren wird eine Lösung aus 5 ml Dichlormethan und 1.0 mmol der in Tabelle 1 genannten primären Diamine (N,N-Ligand) zugetropft. Nach 1 Stunde wird das Reaktionsgemisch bei ca. 600 Pa auf ca. ein Drittel des Volumens eingeengt. Erfolgt keine spontane Kristallisation der Produkte, werden 60 ml Diethylether zugegeben, wobei das Produkt innerhalb 3 Stunden als fester Niederschlag anfällt. Das Produkt wird unter Argon abgesaugt, dreimal mit Diethylether gewaschen und ca. 16 Stunden bei 0.1 Pa getrocknet. Die Farbe sowie deren Elementaranalyse sind in Tabelle 1 aufgeführt.

Beispiel 7: Herstellung von

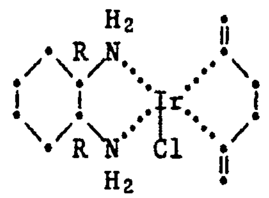

Unter Argon-Schutzgas werden 0,327 g (0,365 mMol) Di-μ-chlortetrakis(cycloocten)diiridium(I) in 25 ml Benzol gelöst. Bei 10°C werden 2,5 ml 1,5-Hexadien zugegeben. Nach 30 Min. Rühren wird 0,8 mMol (-)R,R-1,2-Diaminocyclohexan zugetropft. Nach 1 Stunde Rühren werden 60 ml Petroläther (60°-80°C) zugegeben und das Gemisch 16 Stunden bei 0°C gehalten. Das Produkt fällt als feines gelbes Pulver aus. Es wird mit Petroläther (60°-80°C) gewaschen und 16 Stunden bei 0.1 Pa getrocknet.

Farbe: gelb

Mikroanalyse (Formel $C_{12}H_{24}N_2ClIr$):

| | C | H | N | Cl |
|---|---|---|---|---|
| Berechnet: | 33.99 | 5.71 | 6.61 | 8.36 |
| Gefunden: | 33.4 | 5.9 | 5.7 | 7.4 |

Tabelle 1

| Beispiel Nr. | Komplex | N,N (absolute Konfiguration) | Farbe | Elementaranalyse [Gew.%] |
|---|---|---|---|---|
| 1 | $[Ir(N,N)(COD)]BF_4$ | 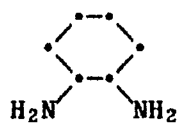 (R,R) | gelb | Gef.: C 32,25; H 5,04; N 5,40; F 15,00<br>Ber.: C 32,37; H 5,43; N 5,39; F 14,63 |
| 2 | " | 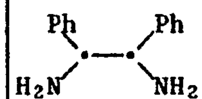 (R,R) | gelb | Gef.: C 40,83; H 4,40; N 4,40; Ir 29,6<br>Ber.: C 41,58; H 5,08; N 4,41; Ir 30,2 |
| 3 | " | 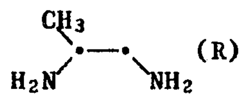 (R) | zitronengelb | Gef.: C 28,55; H 4,70; N 6,13; Ir 40,0<br>Ber.: C 28,64; H 4,81; N 6,07; Ir 41,6 |
| 4 | " | 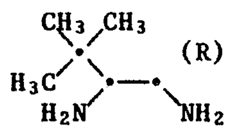 (R) | grünlich-beige | Gef.: C 32,20; H 5,46; N 5,36; F 14,84<br>Ber.: C 31,70; H 5,84; N 5,28; F 14,33 |
| 5 | " | 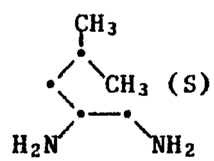 (S) | grünlich-beige | Gef.: C 31,62; H 5,46; N 5,21; F 14,42<br>Ber.: C 31,70; H 5,89; N 5,28; F 14,33 |
| 6 | " | 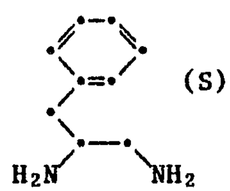 (S) | gelblich-beige | Gef.: C 35,39; H 4,84; N 4,89; Ir 33,4<br>Ber.: C 35,61; H 5,27; N 4,89; Ir 33,52 |

Beispiel 8 (Anwendungsbeispiel)

65,6 mg des gemäss Beispiel 1 hergestellten Komplexes werden unter Sauerstoffausschluss (Argonatmosphäre) in 38,5 ml Isopropanol gelöst. Nach 1 Stunde Rühren bei 60° C werden 6,16 ml 0,1

normale Natriumhydroxid-Lösung zugegeben. Es wird eine weitere Stunde bei 60° C gerührt und anschliessend eine Lösung aus 38,5 ml Isopropanol und 2,28 g Butyrophenon unter Sauerstoffausschluss hinzugefügt. Das molare Verhältnis Substrat zu Katalysator beträgt somit [S]/[Kat.] = 100, die Katalysatorkonzentration $2x10^{-3}$ Mol/l.

Nach 4 Stunden bei 60° C wird gaschromatographisch (OV 101, 120° C, isotherm) eine Ausbeute an 1-Phenyl-1-butanol von 91,0 % bestimmt.

Zur Bestimmung des Enantiomerengehalts nach Mosher wird eine Probe (ca. 0,5 ml) weitgehend vom Lösungsmittel befreit und bei 0° C mit 50 µl optisch reinem α-Methoxy-α-trifluormethylphenylacetylchlorid sowie 0,25 ml trockenem Pyridin versetzt. Nach 15 Minuten wird 30 Minuten auf 70° C erwärmt, nach Abkühlen mit 3 ml 10 prozentiger Citronensäurelösung versetzt und mit Aether die diastereomeren Ester extrahiert. Gaschromatographisch (Kapillarsäule CW 20, 190° C, isotherm) wird ein Enantiomerenüberschuss an (S)-1-Phenylbutanol von ee = 44,6 % bestimmt.

**Patentansprüche**

1. Optisch aktive Iridiumkomplexe der Formel I

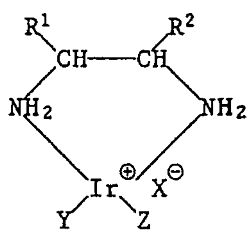

(I),

worin die

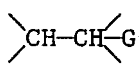-Gruppe

mindestens ein chirales C-Atom enthält, $R^1$ und $R^2$ je Phenyl oder $R^1$ und $R^2$ zusammen unsubstituiertes oder durch $C_1$-$C_4$-Alkyl substituiertes $-(CH_2)_4-$ bedeuten, oder $R^1$ für $C_1$-$C_{12}$-Alkyl, unsubstituiertes oder durch $C_1$-$C_4$-Alkyl substituiertes Cycloalkyl mit 5 oder 6 Ring-C-Atomen, $C_6$-$C_{10}$-Aryl oder $C_7$-$C_{12}$-Aralkyl oder $C_8$-$C_{14}$-Alkaralkyl und $R^2$ für H stehen, $X^{\ominus}$ das Anion einer einbasischen anorganischen oder organischen Säure ist, und Y und Z je Ethylen oder Y und Z zusammen ein offenkettiges oder cyclisches Dien mit 6 bis 10 C-Atomen, dessen Diengruppen über 1 oder 2 C-Atome verbunden sind, bedeuten.

2. Iridiumkomplexe der Formel I gemäss Anspruch 1, worin $X^{\ominus}$ $CF_3SO_3^{\ominus}$, $ClO_4^{\ominus}$, $BF_4^{\ominus}$ oder $PF_6^{\ominus}$ ist.

3. Iridiumkomplexe der Formel I gemäss Anspruch 1, worin Y und Z Ethylen oder Y und Z zusammen 1,5-Cyclooctadien, Norbornadien oder 1,5-Hexadien bedeuten.

4. Iridiumkomplexe der Formel I gemäss Anspruch 1, worin $R^1$ und $R^2$ Phenyl oder zusammen $-(CH_2)_4-$ bedeuten.

5. Iridiumkomplexe der Formel I gemäss Anspruch 1, worin $R^1$ $C_1$-$C_4$-Alkyl, Cyclopentyl, Cyclohexyl, Phenyl, Benzyl, α-Phenylethyl oder Methylbenzyl ist.

6. Iridiumkomplexe der Formel I gemäss Anspruch 1, worin $R^1$ als Alkyl, Methyl, Ethyl, n-Propyl, i-Propyl, n-Butyl, i-Butyl oder t-Butyl ist.

7. Iridiumkomplexe der Formel I gemäss Anspruch 1, worin $X^{\ominus}$ für $BF_4^{\ominus}$ und Y und Z zusammen für Cycloactadien stehen.

8. Verwendung von optisch aktiven Iridiumkomplexen der Formel I gemäss Anspruch 1 als enantioselektive homogene Katalysatoren bei der Transferhydrierung prochiraler Ketone mit sekundären Alkoholen.

**9.** Verfahren zur Herstellung von Iridiumkomplexen der Formel I gemäss Anspruch 1, dadurch gekennzeichnet, dass man $[(Acetonitril)_2(YZ)]IrX$, worin X, Y und Z die in Anspruch 1 angegebene Bedeutung haben, mit einem optisch aktiven Diamin der Formel II

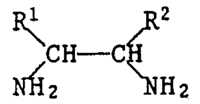

(II),

worin $R^1$ und $R^2$ die zuvor angegebene Bedeutung haben, umsetzt.

## Claims

**1.** An optically active iridium complex of the formula I

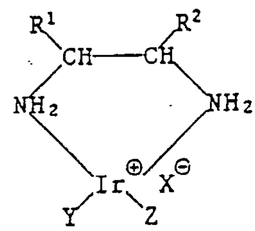

(I),

in which the

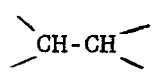

group contains at least one chiral C atom, $R^1$ and $R^2$ are each phenyl or $R^1$ and $R^2$ together are unsubstituted or $C_1$-$C_4$alkyl-substituted $-(CH_2)_4-$ or $R^1$ is $C_1$-$C_{12}$alkyl, unsubstituted or $C_1$-$C_4$alkyl-substituted cycloalkyl having 5 or 6 ring C atoms, $C_6$-$C_{10}$aryl or $C_7$-$C_{12}$-aralkyl or $C_8$-$C_{14}$alkaralkyl and $R^2$ is H, $X^\ominus$ is the anion of a monobasic inorganic or organic acid, and Y and Z are each ethylene or Y and Z together are an open-chain or cyclicdiene having 6 to 10 C atoms whose diene groups are bonded via 1 or 2 C atoms.

**2.** An iridium complex of the formula I according to claim 1, in which $X^\ominus$ is $CF_3SO_3^\ominus$, $ClO_4^\ominus$, $BF_4^\ominus$ or $PF_6^\ominus$.

**3.** An iridium complex of the formula I according to claim 1, in which Y and Z are ethylene or Y and Z together are 1,5-cyclooctadiene, norbornadiene or 1,5-hexadiene.

**4.** An iridium complex of the formula I according to claim 1, in which $R^1$ and $R^2$ are phenyl or together are $-(CH_2)_4-$.

**5.** An iridium complex of the formula I according to claim 1, in which $R^1$ is $C_1$-$C_4$alkyl, cyclopentyl, cyclohexyl, phenyl, benzyl, $\alpha$-phenylethyl or methylbenzyl.

**6.** An iridium complex of the formula I according to claim 1, in which alkyl $R^1$ is methyl, ethyl, n-propyl, i-propyl, n-butyl, i-butyl or t-butyl.

**7.** An iridium complex of the formula I according to claim 1, in which $X^\ominus$ is $BF_4^\ominus$ and Y and Z together are cyclooctadiene.

8. The use of an optically active iridium complex of the formula I according to claim 1 as an enantioselective homogeneous catalyst in the transfer hydrogenation of prochiral ketones with secondary alcohols.

9. A process for the preparation of an iridium complex of the formula I according to claim 1, wherein [-(acetonitrile)$_2$(YZ)]IrX, in which X, Y and Z are as defined in claim 1, is reacted with an optically active diamine of the formula II

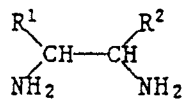

(II),

in which $R^1$ and $R^2$ are as defined above.

**Revendications**

1. Complexes de l'iridium optiquement actifs qui répondent à la formule I :

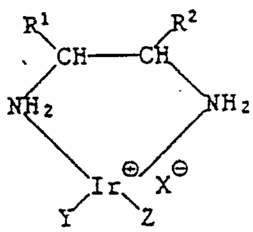

(I)

dans laquelle
le radical

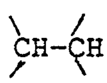

contient au moins un atome de carbone chiral,

$R^1$ et $R^2$ représentent chacun un phényle, ou $R^1$ et $R^2$ représentent, ensemble, un radical $-(CH_2)_4-$ non substitué ou porteur d'un alkyle en $C_1$-$C_4$, ou $R^1$ représente un alkyle en $C_1$-$C_{12}$, un cycloalkyle renfermant 5 ou 6 atomes de carbone dans son cycle, non substitué ou porteur d'un alkyle en $C_1$-$C_4$, un aryle en $C_6$-$C_{10}$, un aralkyle en $C_7$-$C_{12}$ ou un alkyl-aralkyle en $C_8$-$C_{14}$ et $R^2$ représente l'hydrogène,

$X^\ominus$ représente l'anion d'un monoacide organique ou inorganique, et

Y et Z représentent chacun l'éthylène ou, ensemble, un diène en $C_6$-$C_{10}$, à chaîne ouverte ou cyclique, dont les deux radicaux ènes sont reliés par un ou deux atomes de carbone.

2. Complexes de l'iridium de formule I selon la revendication 1 dans lesquels $X^\ominus$ représente $CF_3SO_3^\ominus$, $ClO_4^\ominus$, $BF_4^\ominus$ ou $PF_6^\ominus$.

3. Complexes de l'iridium de formule I selon la revendication 1 dans lesquels Y et Z représentent chacun l'éthylène, ou Y et Z représentent ensemble le cyclooctadiène-1,5, le norbornadiène ou l'hexadiène-1,5.

4. Complexes de l'iridium de formule I selon la revendication 1 dans lesquels $R^1$ et $R^2$ représentent chacun un phényle ou forment ensemble un radical $-(CH_2)_4-$.

5. Complexes de l'iridium de formule I selon la revendication 1 dans lesquels $R^1$ représente un alkyle en $C_1$-$C_4$, un cyclopentyle, un cyclohexyle, un phényle, un benzyle, un phényl-1 éthyle ou un méthylbenzyle.

6. Complexes de l'iridium de formule I selon la revendication 1 dans lesquels $R^1$, en tant qu'alkyle, représente un radical méthyle, éthyle, n-propyle, isopropyle, n-butyle, isobutyle ou tert-butyle.

7. Complexes de l'iridium de formule I selon la revendication 1 dans lesquels $X^\ominus$ représente $BF_4^\ominus$, et Y et Z représentent ensemble le cyclo-octadiène.

8. Application de complexes de l'iridium optiquement actifs qui répondent à la formule I selon la revendication 1 en tant que catalyseurs homogènes énantio-sélectifs dans l'hydrogénation par transfert de cétones prochirales avec des alcools secondaires.

9. Procédé pour préparer des complexes de l'iridium de formule I selon la revendication 1, caractérisé en ce qu'on fait réagir un composé $[(acétonitrile)_2(YZ)]IrX$, dans lequel X, Y et Z ont les significations qui leur ont été données à la revendication 1, avec une diamine optiquement active répondant à la formule II :

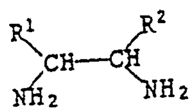

(II)

dans laquelle $R^1$ et $R^2$ ont les significations qui leur ont été données ci-dessus.